# EUROPÄISCHE PATENTSCHRIFT

(11) **EP 1 296 692 B1**
(45) Veröffentlichungstag und Bekanntmachung des Hinweises auf die Patenterteilung: **19.04.2006**
(21) Anmeldenummer: 01960371.1
(22) Anmeldetag: 25.06.2001
(51) Int. Cl.: A61K 35/20, A61P 9/00, A61P 17/06, A61P 1/00

(54) **VERWENDUNG EINER ZUBEREITUNG AUS ODER MIT EINEM GEHALT AN MINDESTENS EINEM DISSIMILIERTEN MILCHSERUM**
USE OF A FORMULATION MADE OF OR CONTAINING AT LEAST ONE DISSIMILATED MILK SERUM
UTILISATION D'UNE PREPARATION CONSTITUEE DE OU CONTENANT UN MINIMUM DE LACTOSERUM DISSIMULE

(30) Priorität: 28.06.2000 DE 10031506; 28.06.2000 DE 10031507; 28.06.2000 DE 10031505; 28.06.2000 DE 10031504; 03.07.2000 DE 10032243; 11.07.2000 DE 10033560; 11.07.2000 DE 10033559; 11.07.2000 DE 10033558; 20.02.2001 DE 10107859
(43) Veröffentlichungstag der Anmeldung: 02.04.2003
(73) Patentinhaber: Sericausa B.V., 6718 Ede, Gelderland (NL)
(72) Erfinder: LEITHE, Ulrich, D-14163 Berlin (DE); ZIJERVELD, Adrianus, Mattheus, Maria, NL-3451 Vleuten (NL)
(74) Vertreter: Winkler, Andreas Fritz Ernst
(86) Internationale Anmeldenummer: PCT/EP2001/007202
(87) Internationale Veröffentlichungsnummer: WO 2002/000231

(56) Entgegenhaltungen:
- EP-A- 0 357 162
- WO-A-98/31377
- DE-A- 4 427 705
- FR-A- 2 718 752
- KAR T., ET AL.: "Influence of fermented whey drink microflora on digestion of lactose" CURRENT SCIENCE, Bd. 75, Nr. 5, 10. Mai 1998 (1998-05-10), Seiten 500-503, XP001022406
- MACEDO R.F., ET AL.: "Production of low cost beverage with soya milk, buffalo cheese whey and cow milk fermented by Lactobacillus casei shirota and Bifidobacterium adolescentis" JOURNAL OF SCIENTIFIC & INDUSTRIAL RESEARCH, Bd. 57, Nr. 10-11, 10. - 11. August 1998, Seiten 679-685, XP001022306

## Beschreibung

Die Erfindung betrifft eine neuartige Verwendung einer Zubereitung aus oder mit einem Gehalt an mindestens einem dissimilierten Milchserum.

EP 0 357 162 B1 offenbart ein Verfahren zur Modifizierung von Molke, bei dem die Molke durch Vergärung mittels geeigneter Milchsäurebakterien zumindest teilweise vom Milchzucker befreit wird. Derart umgesetzte Molke kann in Reinigungsprodukten, Körperreinigungsmitteln, Körperpflegeprodukten wie Cremes, Lotionen, Salben, etc. oder pharmazeutischen Präparaten verwendet werden. Als Beispiel für die Verwendung der modifizierten Molke in der Pharmazie werden Präparate zur Verbesserung, Erhaltung und/oder Wiederherstellung des Immunsystems der menschlichen Haut und Schleimhaut genannt. Konkrete medizinische Indikationen werden nicht angegeben.

Bislang wurden zur Behandlung von Psoriasis, Ichtyosis, atopischem und orthoergischem Ekzem, Akne und ätiologisch ähnlichen Hauterkrankungen verschiedenste Ansätze verfolgt.

Bei den genannten Hauterkrankungen kommen neben einer Lichttherapie, d.h. der Bestrahlung mit ultraviolettem Licht, vor allem Cremes und Salben zum Einsatz, die Corticosteroide enthalten. Corticosteroide sind Hormone und haben insbesondere bei einer Langzeitanwendung vielfältige negative Nebenwirkungen, z.B. ein Dünnerwerden der Haut und Durchblutungsstörungen. Zudem erfordert die Verwendung der Corticosteroide eine stetige Steigerung der Dosis und/oder Stärke der Medikamente, um die Erkrankung im Griff zu behalten. Erkrankungen wie Psoriasis gelten mit bisherigen Medikamenten als nicht heilbar.

Da bei den oben genannten Krankheiten der Einsatz von verschiedenen Medikamenten häufig starke Nebenwirkungen mit sich bringt, besteht daher ein Bedürfnis nach Bereitstellung einer Alternative zur bisherigen Behandlungsweise dieser Krankheiten, die diese Nachteile vermeidet.

Erfindungsgemäß wird diese Aufgabe durch die Verwendung einer Zubereitung aus oder mit einem Gehalt an mindestens einem dissimilierten Milchserum gemäß Anspruch 1 gelöst.

Es ist erfindungsgemäß auch vorgesehen, daß die Zubereitung in Kombination, gleichzeitig oder aufeinanderfolgend, mit für die betreffende Indikation einsetzbaren Wirkstoffen verwendet wird.

Als Ausgangsprodukt für die Herstellung des dissimilierten Milchserums kann vorzugsweise ein Milchserum eingesetzt werden, das aus Kuhmilch, Schafsmilch, Ziegenmilch, Pferdemilch und/oder Kamelmilch gewonnen ist.

Bevorzugt wird Molke verwendet, die als Nebenprodukt der Käse-, Quark- oder Joghurtherstellung gewonnen ist.

Das dissimilierte Milchserum wird vorzugsweise durch Vergärung mit einem oder mehreren Mikroorganismen erhalten, die aus der Gruppe ausgewählt sind, die aus Lactobazillen, Leuconostoc, Streptokokken und anderen Milchsäurebakterien besteht.

Unter Milchserum wird im Zusammenhang der vorliegenden Anmeldung eine nach zumindest teilweiser Abtrennung von Fett und Casein aus Milch gewonnene Flüssigkeit verstanden. Eine Form von Milchserum ist die bei der Käse-, Quark- oder Joghurtherstellung als Nebenprodukt anfallende Molke.

Unter Dissimilation im Sinne der vorliegenden Anmeldung werden allgemein Verfahren verstanden, bei denen höhermolekulare Substanzen unter Energiegewinn in niedermolekulare Substanzen umgewandelt werden. Ein Beispiel hierfür ist die Vergärung durch Mikroorganismen.

Völlig überraschend hat sich ergeben, daß dissimilierte Milchseren - allein oder als Bestandteil einer geeigneten Zubereitung - hervorragend geeignet sind, die Toleranz des menschlichen oder tierischen Organismus, einschließlich Mikroorganismen, optimal einzustellen. Es hat sich gezeigt, daß dies in vielfältiger Weise zur Prävention, Prophylaxe oder Therapie von Erkrankungen genutzt werden kann, aber auch allgemein für die Stärkung und Kräftigung des Organismus. Im Speziellen hat sich gezeigt, daß dissimilierte Milchseren erfolgreich zur Behandlung der obengenannten Hauterkrankungen und Hautinfektionen eingesetzt werden können.

Ohne an diese Theorie gebunden sein zu wollen, wird es gegenwärtig für wahrscheinlich erachtet, daß die Heilerfolge mit dissimiliertem Milchserum im Zusammenhang mit der nunmehr nachgewiesenen Verbesserung bzw. Optimierung der Toleranz des Organismus stehen. Eine gesteuerte Dissimilation von Milchseren liefert offensichtlich ausgewogene Metaboliten-Komplexsubstrate, mit denen die obengenannten Wirkungen erreicht werden können.

Die erfindungsgemäß verwendete Zubereitung wird dabei vorzugsweise aus Milchserum hergestellt, das aus der Milch von Kuh, Schaf, Ziege, Pferd, Kamel oder anderen Säugetieren gewonnen wird. Insbesondere geeignet ist Molke, die bei der Käse- oder Quarkherstellung anfällt, die über Ansäuerung und/oder den Zusatz von Lab abläuft.

Es sei darauf hingewiesen, daß die Gewinnung von Milchserum nicht auf den traditionellen Prozeß der Käseherstellung beschränkt ist, sondern jedes Verfahren eingesetzt werden kann, mit dem Fett und Casein zumindest teilweise, bevorzugt weitgehend, besonders bevorzugt vollständig aus Milch abgetrennt werden.

Das gegenwärtig bevorzugte Verfahren zur Dissimilation von Milchserum ist die mikrobielle Umsetzung, insbesondere die Vergärung mit einem oder mehreren Mikroorganismen unter anaeroben oder mikroaerophilen Bedingungen. Für die mikrobielle Dissimilation von Milchserum eignen sich insbesondere Lactobazillen, wie etwa Lactobacillus acidophilus, Lactobacillus bulgaricus, Lactobacillus lactis, Lactobacillus helveticus und Lactobacillus caseii, Leuconostoc, Streptokokken, wie etwa Streptococcus lactis und Streptococcus thermophilus, sowie andere Milchsäurebakterien, aber auch Hefen und andere Pilze.

Die Vergärungstemperatur und die Dauer des Vergärungsprozesses können je nach den gewünschten Eigenschaften des dissimilierten Milchserums und der eingesetzten Ausgangsstoffe und Mikroorganismen in einem bestimmten Bereich variiert werden. Die bevorzugte Vergärungstemperatur liegt üblicherweise in einem Bereich von 25 bis 40°C und die Dauer der Vergärung beträgt bevorzugt 1 bis 30 Tage.

Selbstverständlich sind auch andere Verfahren zur Dissimilation von Milchserum denkbar und für die Zwecke der vorliegenden Anwendung verwendbar, solange gewährleistet ist, daß der angestrebte Zweck, nämlich die Umwandlung höhermolekularer Substanzen unter Energiegewinn in niedermolekulare Substanzen, erreicht wird. Solche Verfahren können beispielsweise auch auf rein chemischem Wege ablaufen.

Das dissimilierte Milchserum wird für eine erfindungsgemäßen Verwendung vorzugsweise so formuliert, daß es als Zusatz in Lebensmitteln oder Futtermittel verwendet werden kann. Es kann aber, z.B. bei medizinischer Indikation, auch als oral oder in anderer Weise (z.B. intravenös oder topisch) verabreichbares Präparat eingesetzt werden. Dabei kann das dissimilierte Milchserum bzw. das dieses enthaltende Präparat in einigen Fällen allein, in anderen Fällen bevorzugt in Kombination mit anderen Medikamenten für die betreffende medizinische Indikationen verabreicht werden. Insbesondere bei dem Einsatz in Kombination mit anderen Medikamenten hat es sich gezeigt, daß die Dosis letzterer zumindest beträchtlich reduziert werden kann, was insbesondere dann von besonderem Interesse ist, wenn derartige Medikamente signifikante Nebenwirkungen zeigen:

Das nicht-konzentrierte vergärte Milchserum kann unmittelbar und ohne weitere Verarbeitung, d.h. in flüssiger Form, angewendet werden. Obgleich in der fertigen Formulierung noch die verwendeten Mikroorganismen enthalten sein können, kann das dissimilierte Milchserum aber auch pasteurisiert oder sterilisiert verwendet werden. Weiterhin ist es möglich, das dissimilierte Milchserum durch Auflconzentrieren in ein viskoses oder sogar festes Produkt umzuwandeln. Zur Aufkonzentrierung können z.B. Membranfiltrationsverfahren oder das schonende Entziehen von Wasserdampf unter vermindertem Druck bei schonenden Temperaturen, beispielsweise unterhalb von 40°C, angewendet werden.

Das aufkonzentrierte Produkt kann in geeigneter Weise noch weiter getrocknet, z.B. gefriergetrocknet, und ggf. pulverisiert werden. Aus einem entsprechenden Material können mit geeigneten Verfahren Tabletten für die orale Verabreichung hergestellt werden, die ggf. auch magensaftresistent beschichtet sein können.

Die zu verabreichende Dosis an dissimiliertem Milchserum bzw. dieses enthaltendem Präparat kann je nach Einsatzbereich, Krankheitsbild, Alter und Zustand des Patienten, Stadium der Krankheit, ergänzender Medikamentierung, Applikationsweg, Applikationsform, etc. in weitem Bereich variieren. Für den jeweiligen Fachmann ist es jedoch problemlos möglich, eine geeignete Dosierung einzustellen, wobei für die in den Beispielen näher beschriebenen Indikationen die dort angegeben Mengen als Anhaltspunkt dienen können.

### Beispiel 1: Dissimilierung, von Milchserum

Zur Herstellung eines möglichen dissimilierten Milchserums wird die bei der Käseproduktion anfallende Molke aus Kuhmilch unmittelbar in Gärtanks gefüllt. Die Molketemperatur beträgt etwa 30°C, der pH-Wert 5,8 und der Milchzuckergehalt 4,7 %. Diese Molke wird dann mit einer Vorzucht von Lactobacillus acidophilus angeimpft. Die Tanks werden mit Gäraufsätzen verschlossen und bei 30°C für 25 Tage inkubiert.

Das so dissimilierte Milchserum hat einen pH-Wert von 3,4 und einen Milchzuckergehalt von 0,35 % in einem stark milchsauren Milieu. Die Population der Mikroorganismen wird deutlich von Lactobacillus acidophilus dominiert.

Das so hergestellte dissimilierte Milchserum kann ohne weitere Behandlung für die genannten Verwendungszwecke eingesetzt werden. Eine Aufkonzentrierung läßt sich zur Reduktion des Volumens um einen Faktor von 5, bevorzugt 10, durch Entzug des Wassers im Vakuum bei einer Temperatur von maximal 40°C erzielen.

### Beispiel 2: Wirkung der erfindungsgemäßen Zubereitung auf die Toleranz des Organismus

Um die Wirksamkeit der Zubereitung auf die Toleranz des Organismus zu testen, wird ein dissimiliertes Milchserum, wie hergestellt in Beispiel 1, verwendet. Der Einfluß der erfindungsgemäßen Zubereitung wird in vivo am Stratum corneum der menschlichen Haut getestet. Die Messungen erfolgen durch Infrarotspektrometrie. Dazu wird ein Mittelspektrum-Infrarotspektrometer (Biorad Excalibur) mit Flachkristall verwendet. Der Einfallswinkel beträgt 45°.

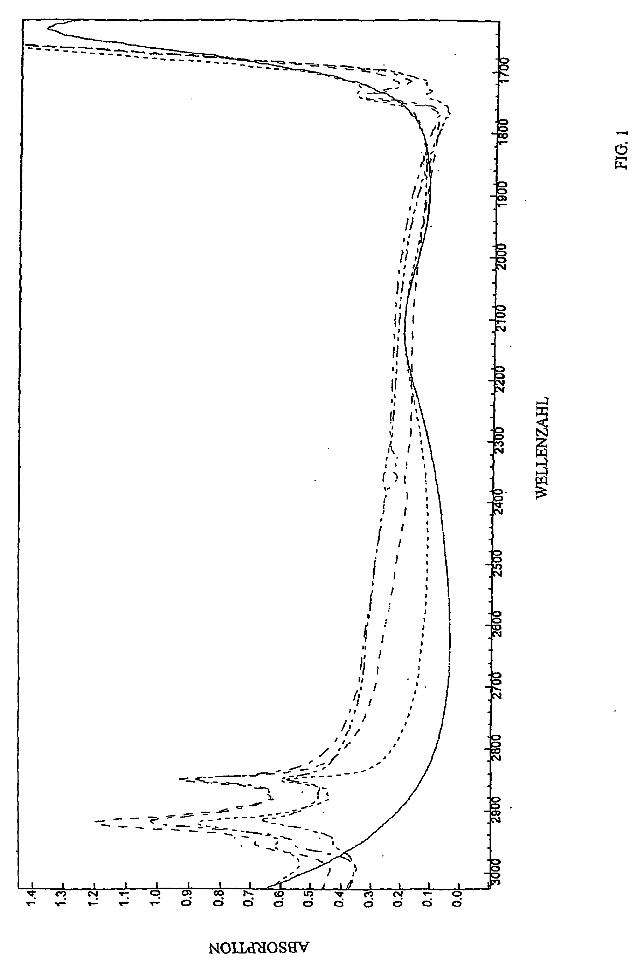

Messungen wurden an insgesamt 20 Probanden (jeweils linker Unterarm) vorgenommen. Die Ergebnisse ergeben sich aus den Figs. 1 bis 4.

Zunächst wurden Messungen an unbehandelter, ungeschädigter Haut vorgenommen. Fig. 1 zeigt beispielhaft die Infrarotspektren von Haut einiger Probanden im Wellenzahlbereich von 3.000 bis 1.600. Die Infrarotspektren sämtlicher getesteten Probanden zeigen in diesem Bereich einen ähnlichen Verlauf, d.h. ausgeprägte Peaks im Bereich 3.000 bis 2.800 sowie ab ca. 1.700. Im Bereich zwischen 2.700 und 1.800 zeigt sich grundsätzlich ein flacher Kurvenverlauf ohne signifikante Peaks.

In einem nächsten Schritt wurde bei den Probanden die minimale Erythemdosis (MED) bestimmt, d.h. die geringste Dosis UV-B-Strahlung, die nach ca. 8 Std. ein Hauterythem bewirkt.

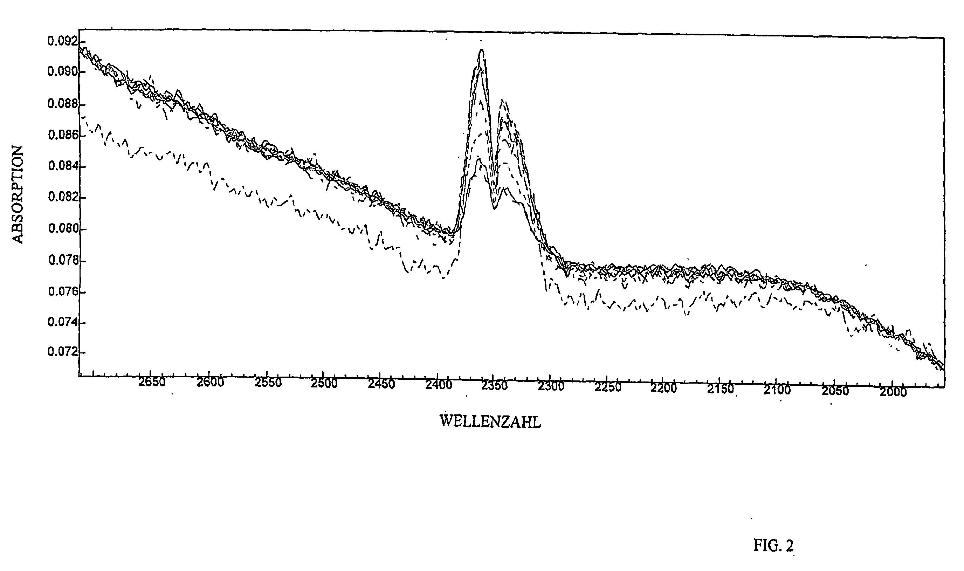

Nach UV-Bestrahlung der Hautstellen mit 70 % MED und erneuter Messung mit dem Infrarotspektrometer zeigte sich im Wellenzahlbereich zwischen 2.450 und 2.250 ein einheitlicher signifikanter Kurvenverlauf, wie er beispielhaft in Fig. 2 dargestellt ist. Die entsprechenden Peaks können der Bildung von cis-Urocansäure aus dem in den Hautzellen vorhandenen trans-Isomer durch UV-Bestrahlung zugeordnet weiden. Es ist bekannt, daß trans-Urocansäure ein natürlicherweise in der Haut gebildeter UV-Absorber ist, der bei UV-Bestrahlung in das entsprechende cis-Isomer umgelagert wird. Es sei darauf hingewiesen, daß der Kurvenverlauf in dem Wellenzahlbereich von Fig. 2 sowohl unmittelbar nach der Bestrahlung als auch noch nach zwei Wochen nahezu identisch ist.

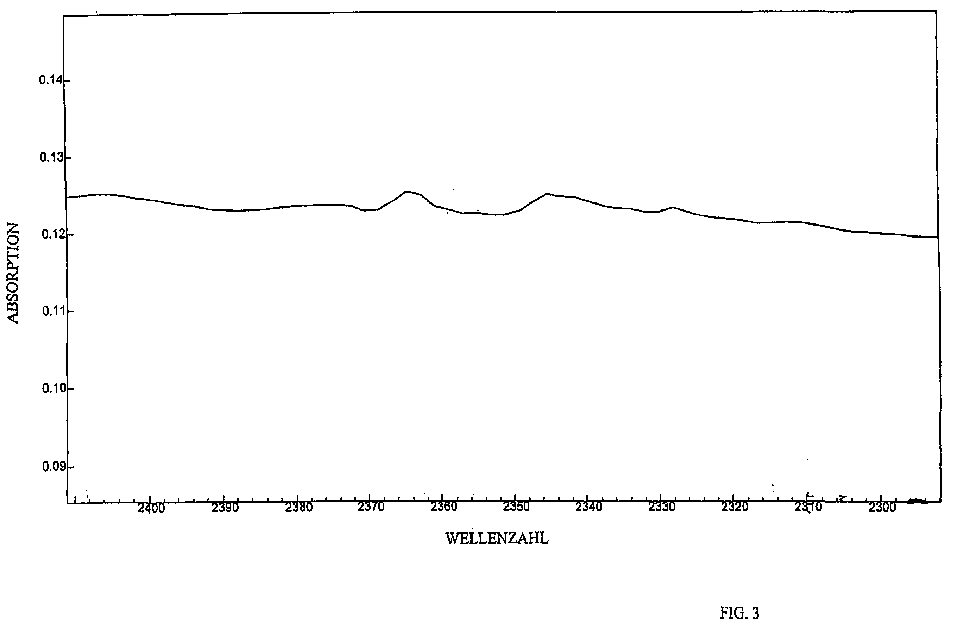

Die bestrahlten Hautstellen wurden bei zehn Probanden zweimal täglich über einen Zeitraum von zwei Wochen behandelt. Eine erneute Messung der behandelten Hautstellen ergab das Spektrum von Fig. 3, in dem zu erkennen ist, daß die Peaks nahezu verschwunden sind, d.h. im wesentlichen eine erneute Abflachung der Kurve zu beobachten ist, was die Folgerung zuläßt, daß die Verhältnisse in der Haut sich durch die Behandlung mit der erfindungsgemäßen Zubereitung wieder normalisiert haben.

Bei sämtlichen Probanden wurde erneut der MED-Wert bestimmt. Während sich bei den Probanden, die nicht mit der erfindungsgemäßen Zubereitung behandelt worden waren, die gleichen MED-Werte ergaben wie vor der ersten UV-Bestrahlung, zeigten sich bei der Probandengruppe, die mit der erfindungsgemäßen Zubereitung behandelt worden war, deutlich höhere MED-Werte, d.h. durch die Behandlung mit der erfindungsgemäßen Zubereitung war die Toleranz der Haut gegenüber UV-Bestrahlung deutlich erhöht.

Die entsprechenden Hautstellen wurden jetzt erneut mit 70 % MED bestrahlt, d.h. im Falle der mit der erfindungsgemäßen Zubereitung behandelten Probanden mit einer absolut gesehen höheren Dosierung. Nach der erneuten UV-Bestrahlung ergaben sich die aus Fig. 4 ersichtlichen Infrarotspektren, wobei die untere, durchgezogene Linie repräsentativ für einen Kurvenverlauf bei Probanden ist, die mit der erfindungsgemäßen Zubereitung behandelt worden waren, während die obere, unterbrochene Linie das Ergebnis bei Probanden zeigt, die nicht mit der erfindungsgemäßen Zubereitung behandelt worden waren.

Während sich bei den nicht-behandelten Probanden im wesentlichen derselbe Kurvenverlauf zeigt wie nach der ersten UV-Bestrahlung, kann man erkennen, daß die Haut von mit der erfindungsgemäßen Zubereitung behandelten Probanden nicht die für UV-Bestrahlung typische Veränderung zeigt. Vielmehr bleibt der Kurvenverlauf im wesentlichen so wie vor der UV-Bestrahlung. Ohne an irgendeine Theorie gebunden sein zu wollen, wird gegenwärtig vermutet, daß mit der erfindungsgemäßen Zubereitung behandelte Haut in der Lage ist, UV-Licht in stärkerem Maße zu tolerieren, ohne daß dabei die Bildung und/oder Umwandlung von trans-Urocansäure eine Rolle spielt. Es sei darauf hingewiesen, daß durch Messungen an der erfindungsgemäßen Zubereitung nachgewiesen werden konnte, daß diese keine Komponenten enthält, die direkt als UV-Absorber anzusehen sind. Die beobachteten Wirkungen sind daher tatsächlich auf eine Erhöhung der Toleranzen in der Haut gegenüber UV-Bestrahlung zurückzuführen. Die Zusammenschau mit den im folgenden genauer beschriebenen Heilerfolgen bei verschiedenen Krankheiten erlaubt den Rückschluß, daß die Verwendung der erfindungsgemäßen Zubereitung zu einer Verbesserung bzw. Optimierung der Toleranz des Organismus führt.

### Beispiel 3: Verwendung der erfindungsgemäßen Zubereitung zur Behandlun von Hauterkrankungen

In allen Beispielen wurde ein Präparat verwendet, das gemäß Beispiel 1 hergestellt werden kann.

### Beispiel 3a

Bei neun Personen mit einem atopischen Ekzem und einer in jungen Jahren durchgemachten Kuhmilchallergie und einem positiven IgE-RAST auf Kuhmilch wurde eine Untersuchung auf eventuelle allergische Hautreaktionen durch Kontakt mit dem Präparat (ohne Konservierungsmittel und Parfüm) durchgeführt. Das Präparat wurde für 48 Stunden unter Okklusion mittels eines Patchtests mit der Haut der Innenseite des Unterarmes der Versuchspersonen in Kontakt gebracht. Bei keiner Versuchsperson konnte nach Beendigung eine allergische Reaktion des Typs IV festgestellt werden. Daraufhin wurde auf der Innenseite des Unterarmes ein "Scratch-Test" durchgeführt. Bei keiner Versuchsperson konnte eine allergische Reaktion des Typs I festgestellt werden.

### Beispiel 3b

Bei acht Personen mit einer chronischen und stabilen Psoriasis auf der Streckseite des Ellenbogens, die vor Beginn der Studie länger als drei Wochen unbehandelt sein sollte, wurde das Präparat für eine Dauer von drei Wochen auf die Streckseite ihres linken Ellenbogens aufgetragen. Der rechte Ellenbogen blieb völlig unbehandelt. Nach einer Woche bemerkte keine Versuchsperson einen vorteilhaften Effekt. Gegen Ende der dritten Woche konnte bei sechs der acht Versuchspersonen eine sichtbare und fühlbare Verbesserung ihrer Hautabweichungen, verglichen mit dem Beginn der Studie und mit ihrem unbehandelten Ellenbogen festgestellt werden.

### Beispiel 3c

Für diese Untersuchung wurden neun Personen mit einem chronischen orthoergischen Ekzem der Hände (sogenannte Hausfrauenhände) ausgesucht, deren orthoergisches Ekzem länger als drei Wochen nicht behandelt worden war, und die instruiert worden waren, ihre täglichen Beschäftigungen unverändert durchzuführen. Alle neun Patienten meldeten innerhalb einiger Tage nach dem Beginn der Behandlung durch topische Applikation des Präparates eine deutliche subjektive und fühlbare Verbesserung. Alle neun Patienten führten die dreiwöchige Studie zu Ende und es konnte festgestellt werden, daß der positive Effekt in diesen drei Wochen unverändert geblieben war. Bei einer dermatologischen Untersuchung konnte nach diesen drei Wochen eine deutliche Abnahme der Schuppung an den Fingern und ein deutlicher Eindruck von einer geschmeidigeren Haut festgestellt werden.

### Beispiel 3d

Neun Personen mit einem stabilen chronischen atopischem Ekzem in den Ellenbogenfalten wurden instruiert, das Präparat drei Wochen lang zweimal täglich auf die linke Ellenbogenfalte aufzutragen und die rechte Ellenbogenfalte unbehandelt zu belassen. Nach einer Woche wurde bei allen Personen festgestellt, daß das Präparat anfänglich ein brennendes Gefühl in der Ellenbogenfalte erzeugte. Nach der dritten Woche konnte bei sieben Personen allmählich ein Verbesserungseffekt festgestellt werden. Sehr auffallend war dabei die Abnahme des Juckgefühls. Diese sieben Personen wollten auch nach der dritten Woche die Behandlung mit dem Präparat fortsetzen.

### Beispiel 3e

Bei sechs Personen mit einem atopischen Ekzem äuf beiden Unterarmen wurde drei, Wochen lang in den ersten drei Tagen der Woche beide Unterarme mit einem Corticosteroid des Grads III behandelt und in den folgenden vier Tagen der Woche mit einer Emulsion (Paraffin liquidum und Vasilin album in gleicher Menge). Der rechte Unterarm sollte dabei immer eine halbe Stunde zuvor mit dem Präparat eingerieben werden. Nach Möglichkeit sollten die vier Tage ohne Behandlung mit Corticosteroiden alle zwei Wochen jeweils um einen. Tag verlängert werden. Dabei zeigte sich, daß nur der mit dem Präparat behandelte Unterarm eine längere Pause zwischen den Corticoapplikationen ermöglicht: bei allen Versuchspersonen nahm diese Zeit von vier über fünf auf sechs Tage zu.

### Beispiel 4: Verwendung der erfindungsgemäßen Zubereitung zur Behandlung von Hautinfektionen

Das Präparat, das gemäß Beispiel 1 hergestellt worden ist, kann auch mit Erfolg bei verschiedensten Arten von Hautinfektionen eingesetzt werden.

## Patentansprüche

1. Verwendung einer Zubereitung aus oder mit einem Gehalt an mindestens einem dissimilierten Milchserum zur Herstellung eines Arzneimittels oder als Zusatzstoff zu Lebensmitteln oder Futtermitteln zur Behandlung, Prävention und Prophylaxe von Psoriasis, Ichtyosis, atopischem und orthoergischem Ekzem, Akne sowie von Hautinfektionen.

2. Verwendung nach Anspruch 1, **dadurch gekennzeichnet, daß** die Zubereitung in Kombination, gleichzeitig oder aufeinanderfolgend, mit für die betreffende Indikation einsetzbaren Wirkstoffen verwendet wird.

3. Verwendung nach Anspruch 1 oder 2, daß als Ausgangsprodukt für die Herstellung des dissimilierten Milchserums ein Milchserum eingesetzt wird, das aus Kuhmilch, Schafsmilch, Ziegenmilch, Pferdemilch und/oder Kamelmilch gewonnen ist.

4. Verwendung nach einem der Ansprüche 1 bis 3, **dadurch gekennzeichnet, daß** Molke verwendet wird, die als Nebenprodukt der Käse-, Quark- oder Joghurtherstellung gewonnen ist.

5. Verwendung nach einem der vorangehenden Ansprüche, **dadurch gekennzeichnet, daß** das dissimilierte Milchserum durch Vergärung mit einem oder mehreren Mikroorganismen erhalten wird, die aus der Gruppe ausgewählt sind, die aus Lactobazillen, Leuconostoc, Streptokokken und anderen Milchsäurebakterien besteht.

## Claims

1. Use of a composition consisting of or having a content of at least one dissimilated whey for the production of a medicament or as an additive to foods or feedstuffs for the treatment, prevention and prophylaxis of psoriasis, ichthyosis, atopic and orthoergic eczema, acne, and also skin infections.

2. Use according to Claim 1, **characterized in that** the composition is used in combination, simultaneously or successively, with active substances which can be used for the indication of interest.

3. Use according to Claim 1 or 2, **characterized in that** as starting product for producing the dissimilated whey, a whey is used which is obtained from cow's milk, sheep's milk, goat's milk, horse's milk and/or camel's milk.

4. Use according to one of Claims 1 to 3, **characterized in that** whey is used which is obtained as a by-product of cheese, quark or yogurt production.

5. Use according to one of the preceding claims, **characterized in that** the dissimilated whey is obtained by fermentation using one or more microorganisms which are selected from the group consisting of lactobacilli, leuconostoc, streptococci and other lactic acid bacteria.

## Revendications

1. Utilisation d'une préparation constituée de ou contenant un minimum de lactosérum dissimulé, pour la fabrication d'une médecine ou comme additif pour produits alimentaires ou fourragers, pour le traitemenfi, la prévention et la prophylaxie du psoriasis, de l'ichtyose, de l'eczéma atopique et orthoergique, de l'acné aussi bien que des infections de peau.

2. Utilisation selon la revendication 1, **caractérisée en ce que**, la préparation s'utilise, simultanément ou successivement, en combinaison avec les substances actives utilisées pour l'indication respective.

3. Utilisation selon la revendication 1 ou 2, **caractérisée en ce qu'**on utilise comme produit initial pour la fabrication du lactosérum dissimulé un lactosérum extrait du lait de vache, lait de mouton, lait de chèvre, lait de jument et/ou lait de chameau.

4. Utilisation selon l'une des revendications 1 à 3, **caractérisée en ce qu'**on utilise un lactosérum extrait comme produit secondaire de la fabrication du fromage, du fromage blanc ou du yagourt.

5. Utilisation selon l'une des revendications antérieures, **caractérisée en ce que**, le lactosérum dissimulé s'obtient par fermentation avec l'un ou plusieurs micro-organismes choisis du groupe consistant en lactobacillus, leuconostoc, streptocoque et autres bactéries d'acide lactique.
